# EUROPEAN PATENT SPECIFICATION

(11) **EP 4 161 505 B1**
(45) Date of publication and mention of the grant of the patent: **03.09.2025**
(21) Application number: 21818356.4
(22) Date of filing: 03.06.2021
(51) Int. Cl.: A61K 31/355, A61K 9/107, A61K 31/353, A61K 31/683, A61K 31/685, A61K 31/714, A61P 1/16, A61K 47/44

(54) **COMPOSITION FOR NORMALISATION OF FATTY LIVER AND METHOD FOR PRODUCING THE SAME**
ZUSAMMENSETZUNG ZUR NORMALISIERUNG DER FETTLEBER UND VERFAHREN ZUR HERSTELLUNG DAVON
COMPOSITION POUR LA NORMALISATION DE LA STÉATOSE HÉPATIQUE ET SON PROCÉDÉ DE PRODUCTION

(30) Priority: 04.06.2020 MY PI2020002852
(43) Date of publication of application: 12.04.2023
(73) Proprietor: Hovid, Berhad, 30010 Ipoh, Perak (MY)
(72) Inventor: HO, David Sue San, 30010 Ipoh Perak (MY); HO, Sarah Lee-Mun, 30010 Ipoh Perak (MY)
(74) Representative: Nederlandsch Octrooibureau
(86) International application number: PCT/MY2021/050043
(87) International publication number: WO 2021/246861

(56) References cited:
- WO-A1-2016/150377
- WO-A1-2017/204618
- WO-A1-2019/075277
- WO-A1-97/03651
- WO-A2-02/26324
- CN-A- 107 320 468
- US-A1- 2016 193 306
- MEDCHOICE: "Phosospholipids and multivitamins", 7 February 2020 (2020-02-07), pages 1 - 1, XP093156943, Retrieved from the Internet <URL:www.medchoicepharma.com_wp-content_uploads_2023_03_PHOSMAX-pdf.pdf>
- CARMELA LOGUERCIO ET AL: "Silybin combined with phosphatidylcholine and vitamin E in patients with nonalcoholic fatty liver disease: A randomized controlled trial", FREE RADICAL BIOLOGY & MEDICINE, ELSEVIER INC, US, vol. 52, no. 9, 5 February 2012 (2012-02-05), pages 1658 - 1665, XP028421061, ISSN: 0891-5849, [retrieved on 20120215], DOI: 10.1016/J.FREERADBIOMED.2012.02.008
- ALAADIN ALAYOUBI ET AL: "Molecular modelling and multisimplex optimization of tocotrienol-rich Self Emulsified Drug Delivery Systems", INTERNATIONAL JOURNAL OF PHARMACEUTICS, ELSEVIER, NL, vol. 426, no. 1, 24 January 2012 (2012-01-24), pages 153 - 161, XP028463455, ISSN: 0378-5173, [retrieved on 20120202], DOI: 10.1016/J.IJPHARM.2012.01.049
- EXCELVITE: "Hepatoprotective effects of tocopherols and tocotrienols in fatty liver disease", 16 December 2015 (2015-12-16), pages 1 - 9, XP093157269, Retrieved from the Internet <URL:https://nutraceuticalbusinessreview.com/hepatoprotective-effects-of-tocopherols-and-tocotrienols-in-fatty-liver-disease-114312>
- ANONYMOUS: "Capsules with Phospholipids and Vitamins", 1 July 2012 (2012-07-01), XP055881075, Retrieved from the Internet <URL:https://www.gnpd.com/sinatra/recordpage/1837237/?utmsource=download&utmmedium=rtf>
- DATABASE Mintel February 2015 (2015-02-01), ANONYMOUS: "Hepatoprotective Capsules", XP055885781, retrieved from GNPD Database accession no. 2944627
- KARL-JOSEF GUNDERMANN, ANN KUENKER, ERWIN KUNTZ, MAREK DRO&ZACUTE;DZIK: "Activity of essential phospholipids (EPL) from soybean in liver diseases", PHARMACOLOGICAL REPORTS, POLSKA AKADEMIA NAUK, INSTYTUT FARMAKOLOGII, KRAKOW, PL, vol. 63, no. 3, 1 May 2011 (2011-05-01), PL , pages 643 - 659, XP055363448, ISSN: 1734-1140, DOI: 10.1016/S1734-1140(11)70576-X
- SHU XIANGBING, ZHANG LI, JI GUANG: "Vitamin E Therapy in Non-Alcoholic Fatty Liver Disease", INTERNATIONAL JOURNAL OF CLINICAL MEDICINE, SCIENTIFIC RESEARCH PUBLISHING, INC., US, vol. 05, no. 03, 1 January 2014 (2014-01-01), US , pages 87 - 92, XP055881073, ISSN: 2158-284X, DOI: 10.4236/ijcm.2014.53016
- DAJANI ASAD, ABUHAMMOUR ADNAN: "Treatment of nonalcoholic fatty liver disease: Where do we stand? an overview", SAUDI JOURNAL OF GASTROENTEROLOGY, WOLTERS KLUWER - MEDKNOW PUBLICATIONS, INDIA, 1 April 2016 (2016-04-01), India, pages 91 - 105, XP055881071, Retrieved from the Internet <URL:https://www.ncbi.nlm.nih.gov/pmc/articles/PMC4817303/pdf/SJG-22-91.pdf> DOI: 10.4103/1319-3767.178527
- ANONYMOUS: "What is vitamin E and what does it do?", NIH NATIONAL INSTITUTES OF HEALTH, OFFICE OF DIETARY SUPPLEMENTS, 18 August 2021 (2021-08-18), XP055881068, Retrieved from the Internet <URL:https://ods.od.nih.gov/pdf/factsheets/VitaminE-Consumer.pdf>

## Description

### FIELD OF INVENTION

This invention relates to a composition and method for producing the composition for oral administration to enhance the normalisation of fatty liver in mammals. More particularly, the composition is suitable for human being with liver problem, more particularly but not limited to non-alcoholic fatty liver disease (NAFLD).

### BACKGROUND OF THE INVENTION

Non-alcoholic fatty liver disease (NAFLD) is characterized by increased fat accumulation as triglycerides in the liver and it is not caused by heavy alcohol use. According to Gastroenterology 1998; 114:842-5 and Hepatology, 2010; 52:1836-46, the *two-hits* and *multiple-hits* hypothesis proposed that the cause of NAFLD relates to obesity, diet and maternal predisposition. Patients with NAFLD and obesity have a relative deficit in energy expenditure and disturbances in lipid and glucose homeostasis due to excess macronutrient intake. These macronutrients such as sucrose and fructose can cause direct injury to the small intestinal wall epithelium. The disruption of small intestine integrity might affect the absorption and processing of vitamins. WO 2019/075277 describes liposomes for use in the treatment of fatty liver disease comprising phospholipids and alpha-tocopherol.

In view of the above, this invention provides a composition with effectively delivered micronutrients and vitamins to the liver and also facilitate hepatic regeneration thereof.

### SUMMARY OF INVENTION

One main aim of the present invention is to provide a drug delivery composition for normalising fatty liver using active ingredients comprising tocotrienols, tocopherols and phospholipids.

At least one of the preceding aims is met, in whole or in part, by the present invention, in which one of the embodiments of the present invention is a drug delivery composition of phospholipids, tocotrienols and tocopherols for oral administration to enhance the normalisation of fatty liver, wherein the amount of phospholipids in the composition is in the range of substantially 4.5 % (w/w) to substantially 20 % (w/w), the amount of the tocotrienols in the composition is in the range of substantially 10 % (w/w) to 35 % (w/w) and tocopherol in the composition is in the range of substantially 2 % (w/w) to substantially 10 % (w/w).

Preferably, the composition further comprises water-soluble vitamin selected from the group consisting of nicotinamide, cyanocobalamine and folic acid.

Preferably, the tocotrienols used comprises alpha tocotrienol in the range of substantially 3.5 % (w/w) to substantially 9 % (w/w) beta tocotrienol in the range of substantially 0.4 % (w/w) to substantially 2.2 % (w/w), gamma tocotrienol in the range of substantially 4.5 % (w/w) to substantially 15.5 % (w/w) and delta tocotrienol in the range of substantially 1 % (w/w) to substantially 7.5 % (w/w).

Preferably, the tocopherol used comprises alpha tocopherol in the range of substantially 2 % (w/w) to substantially 10 % (w/w).

Preferably, the phospholipids used is plant-based phophatidylcholine, phosphatidylethanolamine, phosphatidic acid, phosphoinositides or phosphatidylserine.

Another aim of the present invention is to provide a composition which self-emulsify in the presence of aqueous medium with little agitation so as to improve bioavailability of the active ingredients upon orally administered. More particularly, the composition aforementioned is further modified to obtain a self-emulsifying composition by adding suitable oil, surfactant and co-surfactant.

At least one of the preceding aims is met, in whole or in part, by the present invention, in which one of the embodiments of the present invention is a self-emulsifying composition for oral administration to enhance the normalisation of fatty liver, the self-emulsifying comprising a non-ionic surfactant, a co-surfactant, vegetable oil and active ingredients comprising phospholipids, tocotrienols and tocopherol.

Preferably, the self-emulsifying composition further comprises water-soluble vitamins selected from the group consisting of nicotinamide, cyanocobalamine and folic acid.

Advantageously, the non-ionic surfactant used is polyoxy-35 castor oil.

Advantageously, the co-surfactant used is caprylocaproyl macrogol-8-gylcerides.

Advantageously, wherein the vegetable oil used is palm olein.

Another aim of the present invention is to provide a method for producing a composition comprising phospholipids, tocotrienols and tocopherol that self-emulsifies in the presence of aqueous medium. More particularly, the composition aforementioned is modified by the method so as to obtain the self-emulsifying composition.

At least one of the preceding aims is met, in whole or in part, by the present invention, in which one of the embodiments of the present invention includes the use of a self-emulsifying composition comprising phospholipids, tocotrienols and tocopherols in the manufacturing of a supplement to enhance normalisation of fatty liver, wherein the amount of phospholipids is in the range of substantially 4.5 % (w/w) to substantially 20 % (w/w), the amount of tocotrienols is in the range of substantially 10 % (w/w) to substantially 35 % (w/w) and the amount of tocopherols is in the range of substantially 2 % (w/w) to substantially 10 % (w/w).

At least one of the preceding aims is met, in whole or in part, by the present invention, in which one of the embodiments of the present invention is a method for producing a self-emulsifying composition for oral administration to enhance the normalisation of fatty liver, the method comprising the steps of: mixing a non-ionic surfactant, a co-surfactant and vegetable oil to obtain a first mixture; adding phospholipids into the first mixture and mixing to obtain a homogeneous second mixture; and adding tocotrienols and tocopherols into the second mixture and further mixing thereof until homogeneous.

Preferably, the method further comprises a step of melting beeswax into the vegetable oil, followed by adding there into the first mixture.

Preferably, the method further comprises a step of sieving water-soluble vitamin powder into the second mixture followed by shearing and milling thereof.

### DETAILED DESCRIPTION OF THE INVENTION

The present invention provides a drug delivery composition of phospholipids tocotrienols and tocopherols for oral administration to enhance the normalisation of fatty liver. Additionally, the composition comprises water-soluble vitamins therein to reduce hepatic cholesterol content of fatty liver. More particularly, the composition can be modified into a self-emulsifying composition by adding suitable oil, surfactant and co-surfactant. The self-emulsifying composition can form an emulsion easily with gentle agitation, such as movement of the stomach/intestine. As such, active ingredients in the composition can be absorbed efficiently in the body.

According to the embodiments of the invention, the applicant has found that, by combining or associating tocotrienols and tocopherols with phospholipids, a synergistic normalisation effect can be obtained which reduces the symptoms associated with diseases that are caused by excessive cellular oxidative activity and damaged hepatic membrane, more particularly of NAFLD. More particularly, the composition in the present invention enhances liver protective activity.

Accordingly, the present invention provides, in one embodiment, a composition comprising phospholipids, tocotrienols and tocopherols as the active ingredients in an edible capsule or the like. In some embodiments, water soluble vitamins can be further added as active ingredients therein to supplement liver health.

The tocotrienols and tocopherols used in the invention can be derived naturally or synthetically. They can be extracted from plants such as palm oil, rice bran oil, flaxseed oil, wheat germ, barley and certain types of nuts and grains but not limited thereto. It was found that the tocotrienols and tocopherols exhibits potent antioxidant activity in microsomal lipid peroxidation hence providing hepatoprotective property. More particularly, the tocotrienols used comprises alpha tocotrienol in the range of substantially 3.5 % (w/w) to substantially 9 % (w/w), beta tocotrienol in the range of substantially 0.4 % (w/w) to substantially 2 % (w/w), gamma tocotrienol in the range of substantially 4.5 % (w/w) to substantially 15.5 % (w/w), delta tocotrienol in the range of substantially 1 % (w/w) to substantially 7.5 % (w/w). The tocopherol used comprises alpha tocopherol in the range of substantially 2 % (w/w) to substantially 10 % (w/w).

Another active ingredient used in the composition is phospholipids, more preferably plant-based phospholipids. Amongst others, the phospholipids used is phophatidylcholine, phosphatidylethanolamine, phosphatidic acid, phosphoinositides or phosphatidylserine. In the preferred embodiments, the phospholipids used are derived from soy bean, such as soy bean lecithin. It was found that phospholipids can be incorporated into damaged sections of membrane thereby facilitates hepatic regeneration and replaces endogenous, unsaturated phospholipids. Moreover, phospholipids can improve membrane fluidity and possess antioxidative, antiinflammatory, apoptosis-modulating and anti-fibriotic properties.

In some embodiments, water-soluble vitamin selected from the group consisting of nicotinamide, cyanocobalamine and folic acid are used. Additional benefits from the water-soluble vitamin can enhance fatty liver normalisation effect of the composition in the present invention. It was found that nicotinamide can increase redox potential, reduce hepatic cholesterol content and substantially block the gain in liver weight. Further, it was found that cyanocobalamine deficiency can induce higher rates of adiposity and accompanied by a change in lipid metabolism pathways. Supplementing cyanocobalamine can avoid the abovementioned changes. Moreover, folic acid can alleviate inflammatory response in liver thereby contribute to hepatoprotective effect.

The active ingredients can be formed into edible capsules which self-emulsify in the presence of aqueous medium with little agitation so as to improve bioavailability thereof upon orally administered. Particularly, the active ingredients can be blended with suitable oil, surfactant and co-surfactant. The present invention uses a combination of oil, surfactant and co-surfactant which enable the active ingredients to be effectively released as well as absorbed by the body system. Particularly, palm olein is used as a carrier. Further, non-ionic surfactant is used due to its self-emulsifying capability. Further, the non-ionic surfactant used is non-toxic and nonreactive to produce a chemically stable system. Further, the co-surfactant used is caprylocaproyl macrogol-8-glycerides. Together with the active ingredients aforementioned, the combination provides a stable emulsion upon slight agitation.

One embodiment of the present invention provides a self-emulsifying composition comprises substantially 0.5 % (w/w) to substantially 50 % (w/w) of palm olein, substantially 1 % (w/w) to substantially 20 % (w/w) of caprylocaproyl macrogol-8-glycerides, substantially 5 % (w/w) to substantially 30 % (w/w) of polyoxy-35 castor oil, substantially 4.5 % (w/w) to substantially 20 % (w/w) of soy bean lecithin substantially 10 % (w/w) to substantially 35 % (w/w) of tocotrienols and substantially 2 % (w/w) to substantially 10 % (w/w) of tocopherol.

The composition in the aforementioned embodiment can be obtained by the mixing palm olein, caprylocaproyl macrogol-8-glycerides, and polyoxy-35 castor oil to obtain a homogeneous first mixture. Soy bean lecithin is further added into the first mixture and further mixing to obtain a homogeneous second mixture. Thereinafter, the tocotrienols and tocopherols is then added into the second mixture and further mixing thereof until homogeneous.

Another embodiment of the present invention provides a composition comprises substantially 0.1 % (w/w) to substantially 10 % (w/w) of beeswax, substantially 0.5 % (w/w) to substantially 50 % (w/w) of palm olein, substantially 1 % (w/w) to substantially 20 % (w/w) of caprylocaproyl macrogol-8-glycerides, substantially 5 % (w/w) to substantially 30 % (w/w) of polyoxy-35 castor oil, substantially 4.5 % (w/w) to substantially 20 % (w/w) of soy bean lecithin, substantially 10 % (w/w) to substantially 35 % (w/w) of tocotrienols, substantially 2 % (w/w) to substantially 10 % (w/w) of tocopherol, substantially 1.5 % (w/w) to substantially 7.5 % (w/w) of nicotinamide, substantially 0.01 % (w/w) to substantially 0.05% (w/w) of cyanocobalamine and substantially 0.02 % (w/w) to substantially 0.1 % (w/w) of folic acid.

The composition in the aforementioned embodiment can be obtained by melting beeswax at 70 °C in a portion of palm olein. Separately, another portion of palm olein is mixed with caprylocaproyl macrogol-8-glycerides and polyoxy-35 castor oil to obtain a homogeneous first mixture. The first mixture is then homogeneously combined with the beewax in palm olein. Soy bean lecithin is further added into the first mixture and further mixing to obtain a homogeneous second mixture. In this embodiment, water-soluble vitamin powders of nicotinamide, cyanocobalamine and folic acid are sieved through 60 mesh and then added into the second mixture, followed by shearing and milling thereof. Thereinafter, the mixture of tocotrienols and tocopherols is further added there into the second mixture, followed by mixing thereof until homogeneous.

Accordingly, the composition derived from the abovementioned method can be encapsulated into both soft and hard capsules. The recommended oral dosage for the composition in the present invention is 2 capsules daily. Nonetheless, it shall be understood the dosage may vary for each individual.

The composition and method in producing the composition achieves an increase in extend of nutrient absorption in the intestine. Additionally, the novel composition shows efficacy in replacing damaged sections of liver membrane hence improving hepatic regeneration thereof.

## Claims

1. A drug delivery composition of phospholipids, tocotrienols and tocopherol for oral administration to enhance the normalisation of fatty liver, wherein the amount of phospholipids in the composition is in the range of 4.5 % (w/w) to 20 % (w/w), preferably the phospholipids used is plant-based phophatidylcholine, phosphatidylethanolamine, phosphatidic acid, phosphoinositides or phosphatidylserine, the amount of tocotrienols is in the range of 10 % (w/w) and 35 % (w/w), preferably the tocotrienols comprise alpha tocotrienol in the range of 3.5 % (w/w) to 9 % (w/w), beta tocotrienol in the range of 0.4 % (w/w) to 2.2 % (w/w), gamma tocotrienol in the range of 4.5 % (w/w) to 15.5 % (w/w), delta tocotrienol in the range of 1 % (w/w) to 7.5 % (w/w), and the amount of tocopherol is in the range of 2 % (w/w) to 10 % (w/w), preferably alpha tocopherol in the range of 2 % (w/w) to 10 % (w/w).

2. The composition according to Claim 1 further comprises water-soluble vitamin selected from the group consisting of nicotinamide, cyanocobalamine and folic acid.

3. The drug delivery composition according to claim 1 or 2, wherein the composition is a self-emulsifying composition comprising a non-ionic surfactant, preferably polyoxy-35 castor oil, a co-surfactant, preferably caprylocaproyl macrogol-8-gylcerides, vegetable oil, preferably palm olein, and active ingredients comprising phospholipids, preferably the phospholipids is a plant-based phophatidylcholine, phosphatidylethanolamine, phosphatidic acid, phosphoinositides or phosphatidylserine, tocotrienols, preferably the tocotrienols comprise alpha tocotrienol in the range of 3.5 % (w/w) to 9 % (w/w), beta tocotrienol in the range of 0.4 % (w/w) to 2.2 % (w/w), gamma tocotrienol in the range of 4.5 % (w/w) to 15.5 % (w/w) and delta tocotrienol in the range of 1 % (w/w) to 7.5 % (w/w) and tocopherol, preferably the tocopherol used comprises alpha tocopherol in the range of 2 % (w/w) and 10 % (w/w).

4. The drug delivery composition according to Claim 3 further comprises water-soluble vitamin selected from the group consisting of nicotinamide, cyanocobalamine and folic acid.

5. A method for producing a self-emulsifying composition according to claim 3 or 4, the method comprising the steps of: mixing a non-ionic surfactant, co-surfactant and vegetable oil to obtain a first mixture; adding phospholipids into the first mixture and mixing to obtain a homogeneous second mixture; and adding a mixture of tocotrienols and tocopherols into the second mixture and further mixing thereof until homogeneous.

6. The method according to Claim 5 further comprises a step of melting beeswax into the vegetable oil, followed by adding there into the first mixture.

7. The method according to Claim 5 or 6 further comprises a step of sieving water-soluble vitamin powder into the second mixture followed by shearing and milling thereof.

8. The method according to claim 7, wherein the water- soluble vitamin used is selected from the group consisting of nicotinamide, cyanocobalamine and folic acid.

9. The method according to any one of Claims 5 to 8, wherein the self- emulsifying composition comprises the amount of phospholipids in the range of 4.5 % (w/w) to 20 % (w/w) and the amount of the tocotrienols in the range of 10 % (w/w) to 35 % (w/w) and tocopherol in the range of 2 % (w/w) to 10 % (w/w).

10. The method according to any one of Claims 5 to 9, wherein the tocotrienols comprise alpha tocotrienol in the range of 3.5 % (w/w) to 9 % (w/w), beta tocotrienol in the range of 0.4 % (w/w) to 2.2 % (w/w), gamma tocotrienol in the range of 4.5 % (w/w) to 15.5 % (w/w) and delta tocotrienol in the range of 1 % (w/w) to 7.5 % (w/w).

11. The method according to any one of Claims 5 to 10, wherein the tocopherol used comprises alpha tocopherol in the range of substantially 2 % (w/w) to substantially 10 % (w/w).

12. The method according to any one of Claims 5 to 11, wherein the non-ionic surfactant is polyoxy-35 castor oil.

13. The method according to any one of Claims 5 to 12, wherein the co-surfactant is caprylocaproyl macrogol-8-gylcerides.

14. The method according to any one of Claims 5 to 13, wherein the vegetable oil is palm olein.

15. The method according to any one of Claims 5 to 14, wherein the phospholipid is plant-based phosphatidylcholine, phosphatidylethanolamine, phosphatidic acid, phosphoinositides or phosphatidylserine.

## Patentansprüche

1. Arzneimittelabgabezusammensetzung aus Phospholipiden, Tocotrienolen und Tocopherol zur oralen Verabreichung zur Verbesserung der Normalisierung der Fettleber, wobei die Menge an Phospholipiden in der Zusammensetzung im Bereich von 4,5 % (w/w) bis 20 % (w/w) liegt, wobei vorzugsweise die verwendeten Phospholipide pflanzliches Phosphatidylcholin, Phosphatidylethanolamin, Phosphatidsäure, Phosphoinositide oder Phosphatidylserin sind, wobei die Menge an Tocotrienolen im Bereich von 10 % (w/w) und 35 % (w/w) liegt, wobei die Tocotrienolen vorzugsweise Alpha-Tocotrienol im Bereich von 3,5 % (w/w) bis 9 % (w/w), Beta-Tocotrienol im Bereich von 0,4 % (w/w) bis 2,2 % (w/w), Gamma-Tocotrienol im Bereich von 4,5 % (w/w) bis 15,5 % (w/w), Delta-Tocotrienol im Bereich von 1 % (w/w) bis 7,5 % (w/w) umfassen und die Menge an Tocopherol im Bereich von 2 % (w/w) bis 10 % (w/w), vorzugsweise Alpha-Tocopherol im Bereich von 2 % (w/w) bis 10 % (w/w), liegt.

2. Zusammensetzung nach Anspruch 1, die ferner wasserlösliches Vitamin umfasst, das aus der Gruppe ausgewählt ist, die aus Nicotinamid, Cyanocobalamin und Folsäure besteht.

3. Arzneimittelabgabezusammensetzung nach Anspruch 1 oder 2, wobei die Zusammensetzung eine selbstemulgierende Zusammensetzung ist, die ein nichtionisches Tensid, vorzugsweise Polyoxy-35-Rizinusöl, ein Co-Tensid, vorzugsweise Caprylocaproyl-Macrogol-8-Glyceride, Pflanzenöl, vorzugsweise Palmolein, und Wirkstoffe umfasst, die Phospholipide umfassen, wobei die Phospholipide vorzugsweise ein pflanzliches Phosphatidylcholin, Phosphatidylethanolamin, Phosphatidsäure, Phosphoinositide oder Phosphatidylserin, Tocotrienolen sind, wobei die Tocotrienolen vorzugsweise Alpha-Tocotrienol im Bereich von 3,5 % (w/w) bis 9 % (w/w), Beta-Tocotrienol im Bereich von 0,4 % (w/w) bis 2,2 % (w/w), Gamma-Tocotrienol im Bereich von 4,5 % (w/w) bis 15,5 % (w/w) und Delta-Tocotrienol im Bereich von 1 % (w/w) bis 7,5 % (w/w) und Tocopherol umfassen, wobei das verwendete Tocopherol vorzugsweise Alpha-Tocopherol im Bereich von 2 % (w/w) und 10 % (w/w) umfasst.

4. Arzneimittelabgabezusammensetzung nach Anspruch 3, die ferner ein wasserlösliches Vitamin umfasst, das aus der Gruppe ausgewählt ist, die aus Nicotinamid, Cyanocobalamin und Folsäure besteht.

5. Verfahren zur Herstellung einer selbstemulgierenden Zusammensetzung nach Anspruch 3 oder 4, wobei das Verfahren die folgenden Schritte umfasst: Mischen eines nichtionischen Tensids, eines Co-Tensids und eines Pflanzenöls, um eine erste Mischung zu erhalten; Hinzufügen von Phospholipiden zu der ersten Mischung und Mischen zum Erhalten einer homogenen zweiten Mischung; und Hinzufügen einer Mischung aus Tocotrienolen und Tocopherolen zu der zweiten Mischung und weiteres Mischen derselben, bis eine homogene Mischung entsteht.

6. Verfahren nach Anspruch 5, das ferner einen Schritt des Einschmelzens von Bienenwachs in das Pflanzenöl und das anschließende Hinzufügen zu der ersten Mischung umfasst.

7. Verfahren nach Anspruch 5 oder 6, das ferner einen Schritt des Siebens von wasserlöslichem Vitaminpulver in die zweite Mischung, gefolgt von dem Scheren und Mahlen derselben umfasst.

8. Verfahren nach Anspruch 7, wobei das verwendete wasserlösliche Vitamin aus der Gruppe ausgewählt ist, die aus Nicotinamid, Cyanocobalamin und Folsäure besteht.

9. Verfahren nach einem der Ansprüche 5 bis 8, wobei die selbstemulgierende Zusammensetzung die Menge an Phospholipiden im Bereich von 4,5 % (w/w) bis 20 % (w/w) und die Menge der Tocotrienole im Bereich von 10 % (w/w) bis 35 % (w/w) und Tocopherol im Bereich von 2 % (w/w) bis 10 % (w/w) umfasst.

10. Verfahren nach einem der Ansprüche 5 bis 9, wobei die Tocotrienolen Alpha-Tocotrienol im Bereich von 3,5 % (w/w) bis 9 % (w/w), Beta-Tocotrienol im Bereich von 0,4 % (w/w) bis 2,2 % (w/w), Gamma-Tocotrienol im Bereich von 4,5 % (w/w) bis 15,5 % (w/w) und Delta-Tocotrienol im Bereich von 1 % (w/w) bis 7,5 % (w/w) umfassen.

11. Verfahren nach einem der Ansprüche 5 bis 10, wobei das verwendete Tocopherol Alpha-Tocopherol im Bereich von etwa 2 % (w/w) bis etwa 10 % (w/w) umfasst.

12. Verfahren nach einem der Ansprüche 5 bis 11, wobei das nichtionische Tensid Polyoxy-35-Rizinusöl ist.

13. Verfahren nach einem der Ansprüche 5 bis 12, wobei das Co-Tensid Caprylocaproyl-Macrogol-8-Glyceride sind.

14. Verfahren nach einem der Ansprüche 5 bis 13, wobei das Pflanzenöl Palmolein ist.

15. Verfahren nach einem der Ansprüche 5 bis 14, wobei das Phospholipid pflanzliches Phosphatidylcholin, Phosphatidylethanolamin, Phosphatidsäure, Phosphoinositide oder Phosphatidylserin ist.

## Revendications

1. Composition d'administration de médicament à base de phospholipides, tocotriénols et tocophérols pour l'administration orale afin d'améliorer la normalisation de la stéatose hépatique, où la quantité de phospholipides dans la composition est comprise entre 4,5 % (p/p) et 20 % (p/p), de préférence les phospholipides utilisés sont la phosphatidylcholine, la phosphatidyléthanolamine, l'acide phosphatidique, les phosphoinositides ou la phosphatidylsérine d'origine végétale, la quantité de tocotriénols est comprise entre 10 % (p/p) et 35 % (p/p), de préférence les tocotriénols comprennent de l'alpha-tocotriénol à raison de 3,5 % (p/p) à 9 % (p/p), du bêta-tocotriénol à raison de 0,4 % (p/p) à 2,2 % (p/p), du gamma-tocotriénol à raison de 4,5 % (p/p) à 15,5 % (p/p), du delta-tocotriénol à raison de 1 % (p/p) à 7,5 % (p/p), et la quantité de tocophérol est comprise entre 2 % (p/p) et 10 % (p/p), de préférence de l'alpha-tocophérol à raison de 2 % (p/p) à 10 % (p/p).

2. Composition selon la revendication 1, comprenant en outre une vitamine hydrosoluble choisie dans le groupe constitué du nicotinamide, de la cyanocobalamine et de l'acide folique.

3. Composition d'administration de médicament selon la revendication 1 ou 2, où la composition est une composition auto-émulsifiante comprenant un tensioactif non ionique, de préférence de l'huile de ricin polyoxy-35, un co-tensioactif, de préférence des caprylocaproyl macrogol-8-glycerides, de l'huile végétale, de préférence de l'oléine de palme, et des ingrédients actifs comprenant des phospholipides, de préférence les phospholipides sont la phosphatidylcholine, la phosphatidyléthanolamine, l'acide phosphatidique, les phosphoinositides ou la phosphatidylsérine d'origine végétale, des tocotriénols, de préférence les tocotriénols comprennent de l'alpha tocotriénol à raison de 3,5 % (p/p) à 9 % (p/p), du bêta tocotriénol à raison de 0,4 % (p/p) à 2,2 % (p/p), du gamma tocotriénol à raison de 4,5 % (p/p) à 15,5 % (p/p), du delta tocotriénol à raison de 1 % (p/p) à 7,5 % (p/p) et du tocophérol, de préférence le tocophérol utilisé comprend de l'alpha-tocophérol à raison de 2 % (p/p) à 10 % (p/p).

4. Composition d'administration de médicament selon la revendication 3, comprenant en outre une vitamine hydrosoluble choisie dans le groupe constitué du nicotinamide, de la cyanocobalamine et de l'acide folique.

5. Procédé de production d'une composition auto-émulsifiante selon la revendication 3 ou 4, le procédé comprenant les étapes suivantes : mélanger un tensioactif non ionique, un co-tensioactif et une huile végétale pour obtenir un premier mélange ; ajouter des phospholipides dans le premier mélange et mélanger pour obtenir un deuxième mélange homogène ; et ajouter un mélange de tocotriénols et de tocophérols dans le deuxième mélange et les mélanger ultérieurement jusqu'à l'homogénéité.

6. Procédé selon la revendication 5, comprenant en outre une étape consistant à faire fondre de la cire d'abeille dans l'huile végétale, puis en y ajouter le premier mélange.

7. Procédé selon la revendication 5 ou 6, comprenant en outre une étape de tamisage de poudre de vitamine hydrosoluble dans le deuxième mélange, suivie d'un cisaillement et d'un broyage de celui-ci.

8. Procédé selon la revendication 7, où la vitamine hydrosoluble utilisée est choisie dans le groupe constitué par le nicotinamide, la cyanocobalamine et l'acide folique.

9. Procédé selon l'une quelconque des revendications 5 à 8, où la composition auto-émulsifiante comprend la quantité de phospholipides comprise entre 4,5 % (p/p) et 20 % (p/p) et la quantité de tocotriénols comprise entre 10 % (p/p) et 35 % (p/p) et du tocophérol à raison de 2 % (p/p) à 10 % (p/p).

10. Procédé selon l'une quelconque des revendications 5 à 9, où les tocotriénols comprennent de l'alpha-tocotriénol à raison de 3,5 % (p/p) à 9 % (p/p), du bêta-tocotriénol à raison de 0,4 % (p/p) à 2,2 % (p/p), du gamma-tocotriénol à raison de 4,5 % (p/p) à 15,5 % (p/p) et du delta-tocotriénol à raison de 1 % (p/p) à 7,5 % (p/p).

11. Procédé selon l'une quelconque des revendications 5 à 10, où le tocophérol utilisé comprend de l'alpha-tocophérol à raison de substantiellement 2 % (p/p) à substantiellement 10 % (p/p).

12. Procédé selon l'une quelconque des revendications 5 à 11, où le tensioactif non ionique est l'huile de ricin polyoxy-35.

13. Procédé selon l'une quelconque des revendications 5 à 12, où le co-tensioactif est des caprylocaproyl macrogol-8 -glycerides.

14. Procédé selon l'une quelconque des revendications 5 à 13, où l'huile végétale est l'oléine de palme.

15. Procédé selon l'une quelconque des revendications 5 à 14, où le phospholipide est la phosphatidylcholine, la phosphatidyléthanolamine, l'acide phosphatidique, les phosphoinositides ou la phosphatidylsérine d'origine végétale.
